# EUROPEAN PATENT SPECIFICATION

(11) **EP 1 169 078 B1**
(45) Date of publication and mention of the grant of the patent: **07.05.2014**
(21) Application number: 00919583.5
(22) Date of filing: 23.03.2000
(51) Int. Cl.: A61M 25/10, A61M 25/09

(54) **DEVICE AND METHOD OF GUIDE WIRE BALLOON INFLATION AND DEFLATION TO PREVENT CEREBRAL EMBOLIZATION DURING CAROTID STENTING**
VORRICHTUNG UND VERFAHREN ZUM AUFBLASEN UND ENTLEEREN EINES BALLONS MIT FÜHRUNGSDRAHT ZUR VERHINDERUNG VON HIRNEMBOLISATION WÄHREND KAROTIDEM STENTING
PROCEDE ET DISPOSITIF DE GONFLAGE ET DE DEGONFLAGE DU BALLONNET D'UN CABLE DE GUIDAGE PERMETTANT D'EVITER UNE EMBOLISATION CEREBRALE LORS DE LA POSE D'UN EXTENSEUR DANS LA CAROTIDE

(30) Priority: 25.03.1999 US 126208 P; 26.03.1999 US 126556 P; 22.03.2000 US 533318
(43) Date of publication of application: 09.01.2002
(73) Proprietor: Gore Enterprise Holdings, Inc., Newark, DE 19714-9206 (US)
(72) Inventor: PARODI, Juan Carlos, Buenos Aires 1428 (AR)
(74) Representative: Shanks, Andrew
(86) International application number: PCT/US2000/007785
(87) International publication number: WO 2000/056391

(56) References cited:
- WO-A-97/44085
- WO-A-98/39046
- US-A- 5 378 236
- US-A- 5 695 468
- US-B1- 6 325 777

## Description

### Technical Field

This invention relates to a device and method for inflating and deflating a balloon used to protect the cerebrum against emboli during carotid stenting, where the balloon is carried on a guide wire and where the inflation can be accomplished without the need of a port on the guide wire. Coaxial systems may be applied over the guide wire, because the outside of the guide wire is of a smooth surface with no ports. The device comprises a guide wire, having a lumen inside the guide wire. The guide wire has one end occluded and the other end open, and has a balloon at the occluded end..The guide wire has two or three orifices, each extending from the exterior of the guide wire to reach the lumen of the guide wire. Thus, the lumen of the guide wire is connected to the inside of the balloon through these orifices. The area of the guide wire with the orifices is covered by a silicon or polyurethane balloon. A core segment is inserted into the open end of the guide wire to the closed end of the guide wire before the orifices on the guide wire are covered by a balloon. The core segment may be removed from the guide wire when the open end of the guide wire is inside a basin full of normal saline mixed with non-ionic contrast media solution. When the core segment is removed, it creates a vacuum inside the lumen of the guide wire which is filled immediately with the solution. Then, the core segment is gently advanced until the balloon is fully distended. Through a locking mechanism, the core segment is kept in position during the time needed for the procedure. Coxaxial systems, such as balloon catheter and stent delivery systems, may be applied over the guide wire. After the procedure which may generate emboli is completed, the core segment is unlocked and retrieved and the baboon is thereby deflated.

### Background of the Invention

Although traditionally conventional open surgery has been used in treatments of vascular diseases, such as stenosis of the carotid artery, nowadays endovascular treatments are gaining acceptance. Endovascular treatments are carried out in the lumen of the vascular duct and have the advantage of being less aggressive than the conventional open surgery posing less risk to the patient because it can be performed under limited local anesthesia and without surgery.

Where there is stenosis of an artery, such as the common carotid artery, the internal carotid artery or the external carotid artery, the vascular wall of the artery is affected by a pathologic narrowing that prevents the blood stream from flowing normally. A common treatment consists of endovascular angioplasty, where an angioplasty balloon is inserted into the lumen of the blood vessel and the angioplasty balloon is expanded in order to expand the area having the stenosis. If necessary, a stent is placed to cover the afflicted area.

Balloon angioplasty and stenting are a rapidly growing field in vascular intervention. The main and dreadful complication of carotid balloon angioplasty and stenting is cerebral embolization. Several studies have shown that the incidence of embolization is high during these procedures.

The problem in this treatment is that emboli can be formed during the course of the procedure which can rapidly reach the brain and cause injury and dearth. Emboli are especially prone to be formed when the angioplasty balloon is expanded or while the stent is placed and expanded.

Investigators, such as Jack Theron, used a balloon to prevent particles generated in the affected area from reaching the brain. United States Patent, 5,833,650 of inventor Imran, entitled Catheter Apparatus and Method for Treating Occluding Vessels issued November 10, 1998. The Imran patent described a system to achieve cerebral protection using a guide wire and inflated balloons, which allowed the advancement over the guide wire of a coaxial system of balloon angioplasty catheters and stent delivery systems. The use of occlusion balloons to protect the brain during carotid balloon angioplasty and stenting (CBAS) was presented publicly by Theron in Milwaukee in 1994.

Difficulty exists in keeping the occlusion balloon inflated and undisturbed when changing the coaxial systems. The ports with valves on a guide wire prevent advancement of coaxial systems, that very often need to be changed. For instance, different balloon diameters may be needed in the procedure. Sometimes, one balloon may need to be replaced with a balloon of a different diameter. Systems to deploy a stent and systems to expand a stent with a balloon also may require coaxial-systems. The presence of a port on the guide wire would obligate the operator to deflate and remove the guide wire every time he needed to exchange the coaxial system, such as, for example, either to use a different balloon diameter catheter or to advance a delivery system for stent deployment

Therefore, there is needed in the art a device and method that allows for the inflation and deflation, without the need of a port on the guide wire, of a balloon carried on a guide wire. There is needed a device and method which allow for the use of coaxial systems to be placed over the guide wire to allow for the use and placement of instrumentation needed during the medical procedure without the necessity of deflating the balloon and removing the guide wire.

International Patent Application WO97/44085 and US Patent 6,325,777 describe low profile catheter arrangements having an expandable member, such as an inflatable balloon, positioned on the distal end of a hollow tubular body. An access opening is provided on the tubular body which permits the expandable member to be inflated by attaching devices to inflate or deflate the balloon. A sealer portion is provided in the lumen which defines an open valve position when the sealer portion is positioned proximally of the access opening and defines a closed valve position when the sealer portion is positioned between the access opening and the balloon.

International Patent Application WO98/39046 describes a catheter apparatus for use in the containment and removal of emboli from blood vessels. An inflatable balloon is mounted on the distal end of a hollow guide wire. The guide wire is received within an intermediate catheter, the annulus between the guide wire and the intermediate catheter defining a fluid passage. The intermediate catheter is received within a main catheter and the annulus between the intermediate catheter and the main catheter defines a fluid passage. An inflatable balloon is mounted on the main catheter and a chamber is defined this balloon and the balloon mounted on the guide wire. In use, a fluid flow is generated in the chamber to remove emboli from the chamber.

US Patent 5,695,468 describes a catheter having an elongate shaft with an inflatable balloon connected to its distal end. A fluid displacement rod is disposed with the shaft and the rod is sealed within the shaft so that, in use, movement of the rod causes a bolus of liquid to move into or out of the balloon.

The present invention recognizes that a balloon situated on a hollow guide wire, where the hollow guide wire permits insertion of a core segment into the lumen of the guide wire, will allow for easy and convenient inflation and deflation of the balloon while permitting coaxial systems to be introduced over the guide wire. Thus, the present invention allows for the rapid exchange of coaxial systems without the need to disconnect or connect ports which in prior art have been attached to the guide wire.

### Summary of the Invention

According to an aspect of the present invention there is provided a device for inflating and deflating a guide wire balloon according to claim 1. Other features of the present invention are described in the dependent claims.

The invention comprises a hollow guide wire with a plurality of orifices extending from the exterior of the guide wire to its lumen, an attached balloon which covers the orifices and a core segment. The core segment may be inserted into the lumen of the guide wire and then removed from the lumen of the guide wire when the open end of the guide wire is inserted in a solution. The core segment may be sized to fit snugly within the lumen of the guide wire and may serve as a piston to inflate the guide wire balloon.

The guide wire is provided with a closed end and an open end which form a tubular body, having an internal lumen. Near the closed end of the guide wire there is a guide wire balloon, which may be expandable against the vascular duct to occlude the blood flow.

The guide wire with attached guide wire balloon have a low profile allowing the insertion and advancement of coaxial balloon catheters and stent delivery systems over the guide wire without disturbing the inflated guide wire balloon.

In use, the operator takes the guide wire with the core segment positioned inside the lumen of the guide wire and with the guide wire balloon in its uninflated position on the guide wire, and inserts the closed end of the guide wire with the attached guide wire balloon into the patient through methods known in the art, such as percutaneously into the femoral artery. Then, the guide wire balloon is advanced through the vessel and positioned at the appropriate location in the patient. The open end of the guide wire is inserted inside a solution. A solution of normal saline mixed with non-ionic contrast media may be used. In particular, a solution consisting of 50% normal saline and 50% non-ionic contrast media may be used. The core segment is removed from the lumen of the guide wire with the open end of the guide wire inserted within the solution. The solution fills the lumen of the guide wire. Then, the core segment is gently advanced toward the guide wire balloon until the guide wire balloon is fully dilated. The guide wire balloon is thus expanded against the vascular walls. Once the guide wire balloon is inflated, the core segment is fixed in position within the guide wire by a locking mechanism which does not increase the profile of the guide wire. Therefore, the advancement of coaxial systems over the guide wire is allowed. Coaxial systems may be exchanged without the need to deflate the guide wire balloon.

### BRIEF DESCRIPTION OF THE DRAWINGS

Figure 1 is a longitudinal cross section of the guide wire with the guide wire balloon in its deflated position.
Figure 2 is a longitudinal cross section of the core segment which has not been inserted into the lumen of the guide wire.
Figure 3 is a longitudinal cross section of the hollow guide wire showing the orifices of the guide wire.

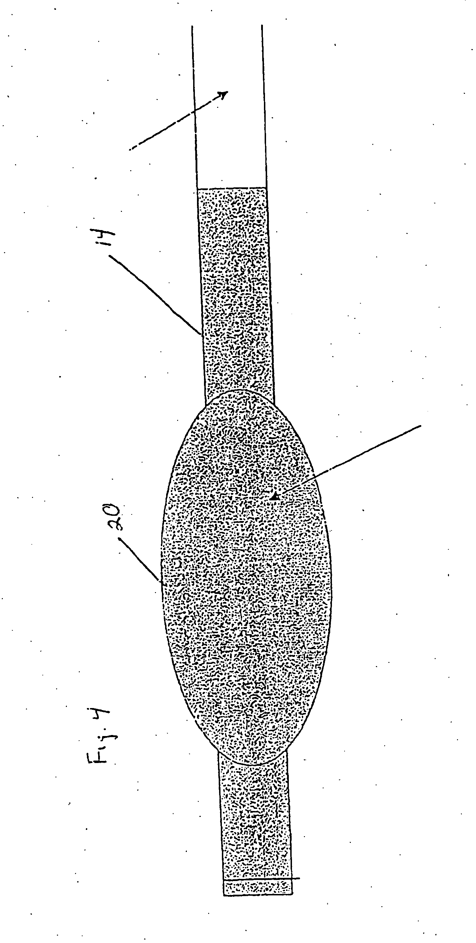

Figure 4 is a longitudinal cross-section of the guide wire showing an inflated guide wire balloon attached thereto.

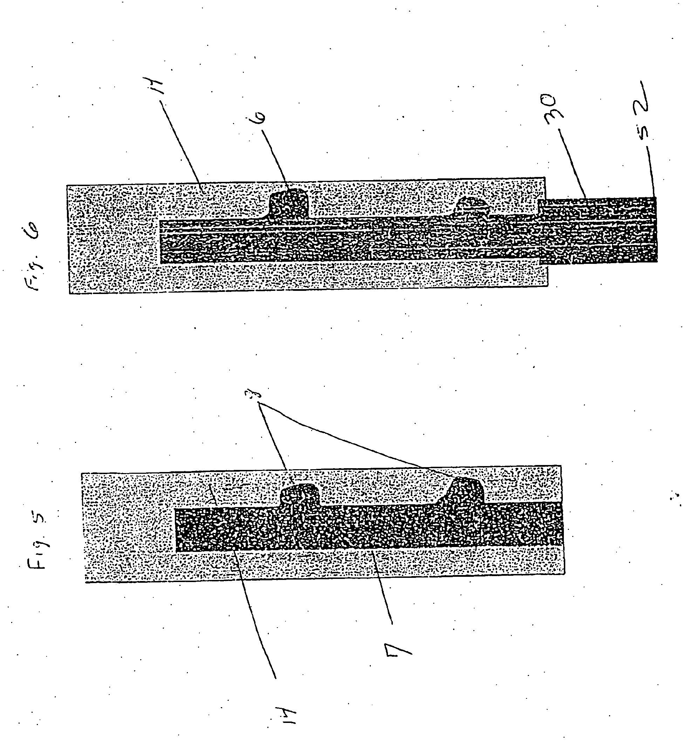

Figure 5 is longitudinal cross-section of the open end of the guide wire showing an embodiment of the guide wire with a groove and two spaces, which are part of a locking mechanism.
Figure 6 is a longitudinal cross-section of a portion of one embodiment of the invention, showing the open end of the guide wire with the core segment inserted into the lumen of the guide wire, with the knob of the core segment engaging one of the housing spaces of the guide wire to immobilize the core segment in its locked position.

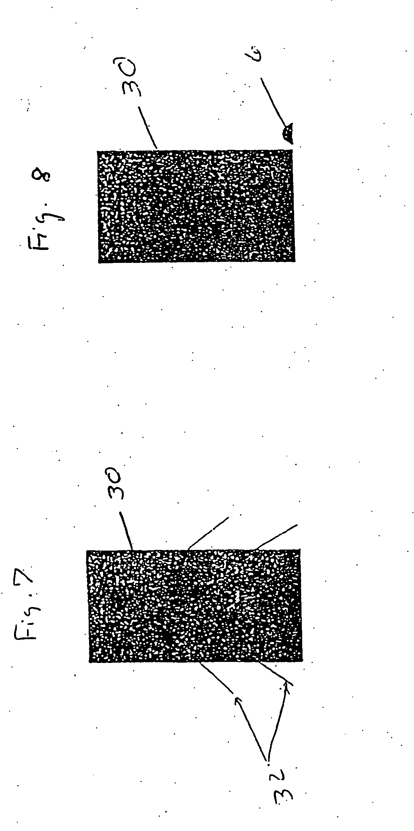

Figure 7 is longitudinal cross-section of the distal end of one embodiment of the core segment, showing three rings, which are a sealing mechanism.
Figure 8 is a longitudinal cross-section of the operator end of one embodiment of the core segment, showing the knob, which is a part of the locking mechanism.

### Description of the Preferred Embodiments

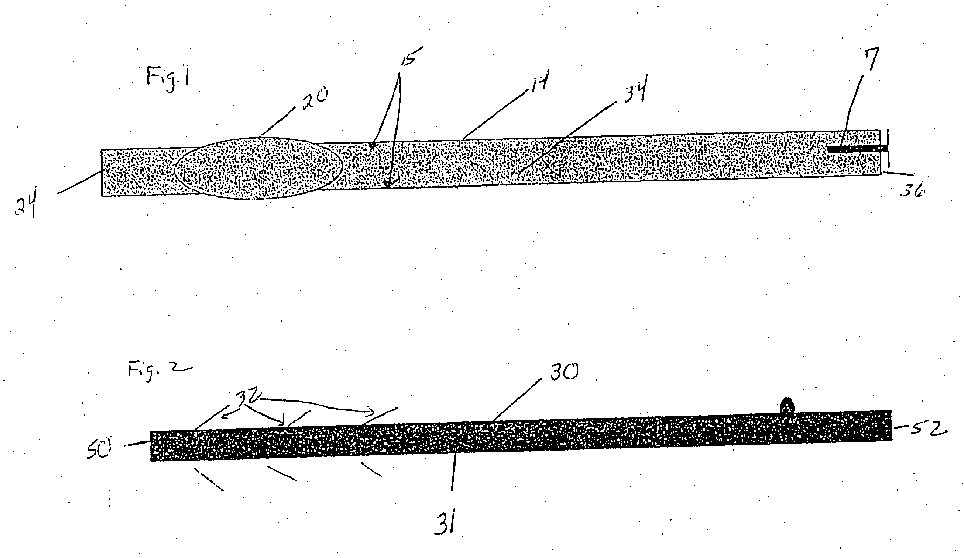

The device of balloon inflation (10) in carotid angioplasty illustrated in FIG. 1 and Fig. 2 comprises a guide wire (14) having a guide wire balloon (20) near the closed end (24) of the guide wire and a core segment (30) which may be inserted into the lumen (34) of the guide wire. The guide wire balloon (20) may be integral with the guide wire (14) and may be formed on the guide wire by means known in the art. The core segment (30) may be moved in an axial direction in the lumen of the guide wire.
In a preferred embodiment for use with carotid stenting, the length of the guide wire is 2.3 meters and the length of the core is 2.4 meters. However, other lengths of the guide wire and core may be used.

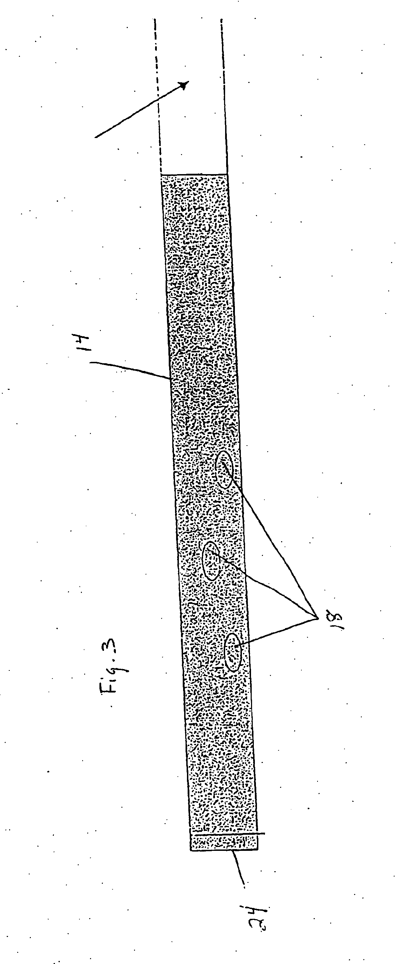

The guide wire (14) comprises a cylinder body (16) having a closed end (24), an open end (36). The cylinder body (16) of the guide wire (14) forms by its inner wall (15) in its interior a lumen (34) of the guide wire. Preferably, the guide wire is made of metal. As shown in Fig. 3, the guide wire (14) has a plurality of orifices (18) near its closed end. In a preferred embodiment the guide wire (14) has two or three such orifices (18). The orifices (18) are openings which extend from the outside of the guide wire (14) into the lumen (34) of the guide wire. Preferably, these orifices (18) are made in different segments of different circumferences of the guide wire. Preferably, these orifices are 1 or 2 mm apart, one from each other. The area of the guide wire with the orifices (18) is covered by the guide wire balloon (20), preferably made of silicon or polyurethane.

Preferably, the core segment (30) is inserted into the guide wire (14) before the guide wire balloon (20) is placed to cover the orifices (18) of the guide wire (14). As shown in Fig. 2, a core segment (30) comprises a solid body (31) and has an operator end (52) and a distal end (50). The core segment (30) corresponds to the lumen of the guide wire. The core segment (30) is inserted into the lumen (34) of the guide wire, so that the distal end (50) of the core segment (30) first enters the lumen (34) of the guide wire. The core segment (30) provides rigidity to the guide wire (14). The core segment (30) is slightly smaller in width than the lumen (34) of the guide wire (14) so as to allow the advancement of the core segment (30) within the lumen (34) of the guide wire (14), but must be of sufficient width so as to not allow seepage of fluid around the core segment (34), when the core segment (34) is properly sealed within the lumen (34) of the guide wire (14) by a sealing mechanism. Such sealing may be accomplished by a variety of means.

The core segment (34) may be fitted with a plurality of rings (32), which attach around the core segment, or the core segment may be covered at its distal end portion with a layer of sealing material, the rings (32) or the sealing material, being made of silicone or other appropriate material, such as TEFLON (tm), to provide a watertight seal of the core segment (34) with the inner wall (15) of the guide wire to prevent leakage of fluid around the core segment (30). The core segment (30) is inserted within the guide wire (14) so as to seal the guide wire (14) in the same way a piston adapts to a cylinder. The core segment (30) is sized in dimension such that there is no noticeable gap between the core segment (30) and the guide wire (14). If the fitting of the core segment (30) within the lumen (34) of the guide wire (14) is not watertight, solution would leak, allowing the guide wire balloon (20) to deflate. Therefore, the core segment (30) must be fitted within the lumen (34) of the guide wire (14), and such fitting may employ the use of seals, rings or the like, so as to be watertight to prevent leakage of fluid around the core segment when the core segment (30) is within the guide wire (14). Rings, such as those made of TEFLON (tm), silicone or the like, may be inserted into the lumen (34) of the guide wire (14) and placed so as to seal the lumen (34) of the guide wire (14) against the core segment (30), when the core segment (30) is within the guide wire (14). Alternatively, as shown in Fig. 2, rings (32), such as those made of TEFLON (tm), silicone or the like, may be placed at the distal end (50) of the core segment (30), so that when the core segment (30) is within the guide wire (14) there is a watertight seal between the sides of the core segment and the inner wall (15) of the guide wire (14). Preferably, two or three rings either within the end of the guide wire (14) or attached to the outside distal end (50) of the core segment (30) may be used to accomplish this watertight seal. Alternatively, the distal end (50) of the core segment (30) may be covered with silicone or TEFLON (tm) to seal the core segment so that no fluid may leak around the core segment (30) when the core segment (30) is positioned within the lumen (34) of the guide wire (14).

Thus, the guide wire (14) has a low profile, because the core segment (30) used to inflate and deflate the guide wire balloon (20) is inserted into the lumen (34) of the guide wire (14) and therefore, does not increase the profile of the guide wire. The guide wire (14) avoids the use of a port for inflation of the guide wire balloon (20), as such port is described in the prior art, which port would increase the profile of the guide wire. Therefore, coaxial systems can be applied over the guide wire (14) of this invention. Typically, balloon catheters and stent delivery systems may be applied, over the guide wire (14) without having to deflate the guide wire balloon.

The guide wire (14) and core segment (30) may be'fitted with a locking mechanism to achieve, when needed, an immobilization of the core segment (30) in relation to the guide wire (14). Such locking mechanism may be achieved without significantly increasing the profile of the guide wire (14). In a preferred embodiment of a locking mechanism, the core segment (30) has a very small prominent knob (6), preferably made of metal or other biocompatible material, which does not extend beyond the outer diameter of the guide wire (14) and which is located near the operator end (52) of the core segment (30). In this embodiment of the locking mechanism, the guide wire (14) has a narrow groove (7), preferably of 3 cm length, which extends longitudinally from the open end (36) of the guide wire (14) toward the closed end (24) of the guide wire (14). The groove (7) has a plurality of side housing spaces (8), preferably 3 cm apart, one to each other. Preferably, there are two or three side housing spaces (8). Preferably, each side housing space (8) is perpendicular to the groove (7) in the guide wire (14). The knob (6) fits within a selected side housing space (8). The core segment (30) is immobilized at the chosen level by rotating the core segment (30) until the knob (6) of the core segment enters the side housing space (6) of the guide wire (14), which thereby locks the core segment in its position within the lumen (34) of the guide wire (14).

In use, after the core segment (30) has been inserted into the open end of the guide wire and positioned, it may be locked and kept immobilized to keep the balloon inflated. The knob (6) of the core segment (30) engages one of the housing spaces (8) of the guide wire (14) when the core segment (30) is in its locked position within the guide wire (14). The mechanism of locking consists of moving the core segment inside the guide wire (14) so as to position the knob (6) at the same point in the circumference as the groove (7) of the guide wire. Once the knob (6) enters the groove (7), attention is paid to the guide wire balloon (14) to determine that it is fully inflated. If the balloon is fully inflated the core segment (30) should be locked in position up to the end of the procedure to keep the balloon fully inflated. The core is locked within the guide wire by a gentle rotation of the core segment (30) to install the knob (6) inside the side housing space (8). After the medical procedure is carried out, the core segment (30) is unlocked and the guide wire balloon (20) is deflated by reversing the rotation of the core segment (30) to release the knob (6) of the core segment from the housing space (8) of the guide wire (14). Thus, the guide wire balloon (20) has a first configuration of reduced size in its uninflated state and a second configuration of expanded size in its inflated state, the guide wire balloon (20) being expandable from its first configuration to it second configuration

In use the guide wire balloon (20) is in its uninflated configuration when the guide wire (14) with guide wire balloon (20) is introduced into the patient. At the appropriate time when the operator chooses to expand the guide wire balloon (20) against the vascular duct to occlude the blood flow the guide wire balloon may be expanded according to the following actions. In carotid stenting, the guide wire may be positioned at the desired location of occlusion, such as in the internal carotid artery beyond the stenosis, before the guide wire balloon (20) is inflated. The open end (36) of the guide wire (14) is inserted inside the desired solution, used to inflate the guide wire balloon, such as a normal saline mixed with non-ionic contrast media. Preferably, a solution consisting of 50% normal saline and 50% non-ionic contrast media is used. The solution may be kept available inside a basin.

The core segment (30) is removed from the lumen (34) of the guide wire (14) with the open end of the guide wire (14) inserted within the saline/contrast media solution. When the core segment (30) is removed it creates a vacuum inside the lumen (34) of the guide wire (14) which is then immediately filled by the solution. The saline/contrast media solution fills the lumen (34) of the guide wire (14). Then, the core segment (30) is gently advanced toward the guide wire balloon (20) until the guide wire balloon (20) is fully dilated. The solution flows through the orifices (18) of the guide wire (14) into the guide wire balloon (20) to inflate the guide wire balloon (20). The guide wire balloon (20) is thus expanded against the vascular walls. The core segment (30) may be locked in the guide wire (14) and kept in position to maintain the inflation of the guide wire balloon (20) during the time needed for the procedure.

After expansion of the guide wire balloon (20), endovascular procedures, such as carotid stenting, may be performed. For example, a catheter, balloon of angioplasty, a stent or any other suitable apparatus or instrument may be passed by way of a coaxial system over the guide wire (14) to reach the appropriate area of the vascular walls for appropriate treatment.

Once the procedure is finished and when the expanded guide wire balloon (20) is no longer needed in its expanded position, the core segment (30) is unlocked and withdrawn from the lumen (34) of the guide wire (14) until the guide wire balloon (20) is completely deflated. Thus, the guide wire balloon may be returned to its uninflated position by removing the core segment (30) from the open end (36) of the guide wire (14).

In particular, a new method for inflating and deflating a guide wire balloon (14), is described below. The operator uses a device of balloon inflation (10), wherein a core segment (30) has been inserted into the open end (36) of the guide wire (14) and positioned at the closed end (24) of the guide wire (14), and the orifices (18) on the guide wire (14) have been covered by a guide wire balloon (20). The operator inserts the closed end (24) of the guide wire (14) into the patient, by means known in the art, such as percutaneously into the femoral artery and then positions the guide wire balloon at the appropriate location of intended use in the body. The core segment (30) may be removed from the guide wire (14) when the open end (36) of the guide wire (14) is inside a basin full of solution, such as normal saline mixed with non-ionic contrast media. When the core segment (30) is removed from the lumen (34) of the guide wire (14), it creates a vacuum inside the lumen (34) of the guide wire which is filled immediately with the solution. Then, the core segment (30) is gently advanced toward the guide wire balloon (20) until the guide wire balloon (20) is fully distended. Through a locking mechanism the core segment (30) is kept in position during the time needed for the procedure. Coaxial systems, such as balloon catheter and stent delivery systems, may be applied over the guide wire (14) without deflating the guide wire balloon (20). After the procedure which may generate emboli, is completed, the core segment (30) is unlocked and retrieved and the guide wire balloon (20) is thereby deflated.

In alternative embodiments of the invention, the methods and apparatus can be used in other procedures and repairs of the anatomy, where there is a need to inflate an occlusion balloon to protect against dangerous emboli. In such procedures, the convenient method and apparatus for the inflation and deflation of a balloon may be likewise be useful.

Reference is further made to supplementary Appendix A, which has additional pages of specification and drawings therein.

Having described this invention with respect to its preferred embodiments, it is to be understood that the description is not meant as a limitation since further variations or modifications may be apparent or may suggest themselves to those skilled in the art. It is intended that the present application cover such variations and modifications as fall/within the scope of the appended claims.

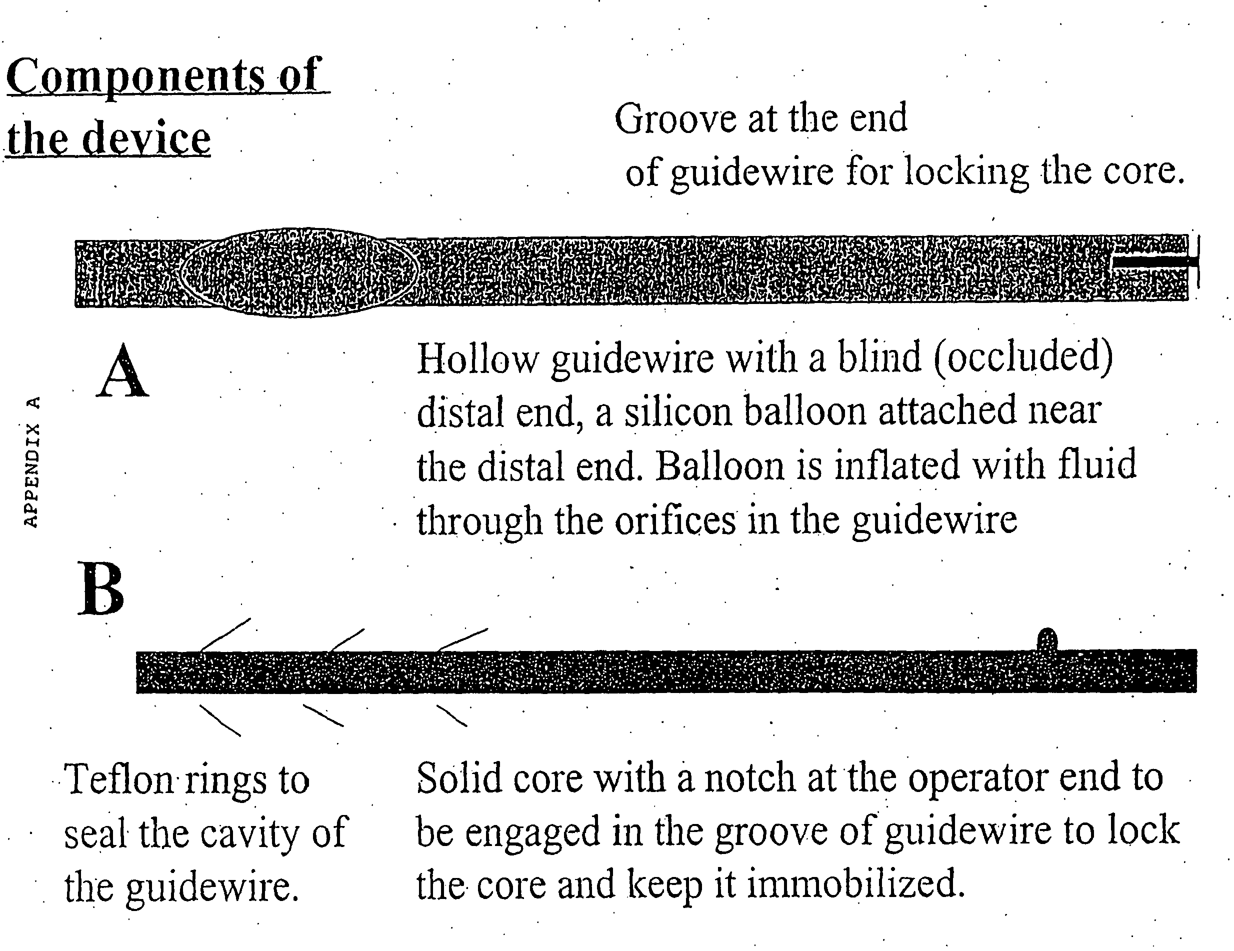

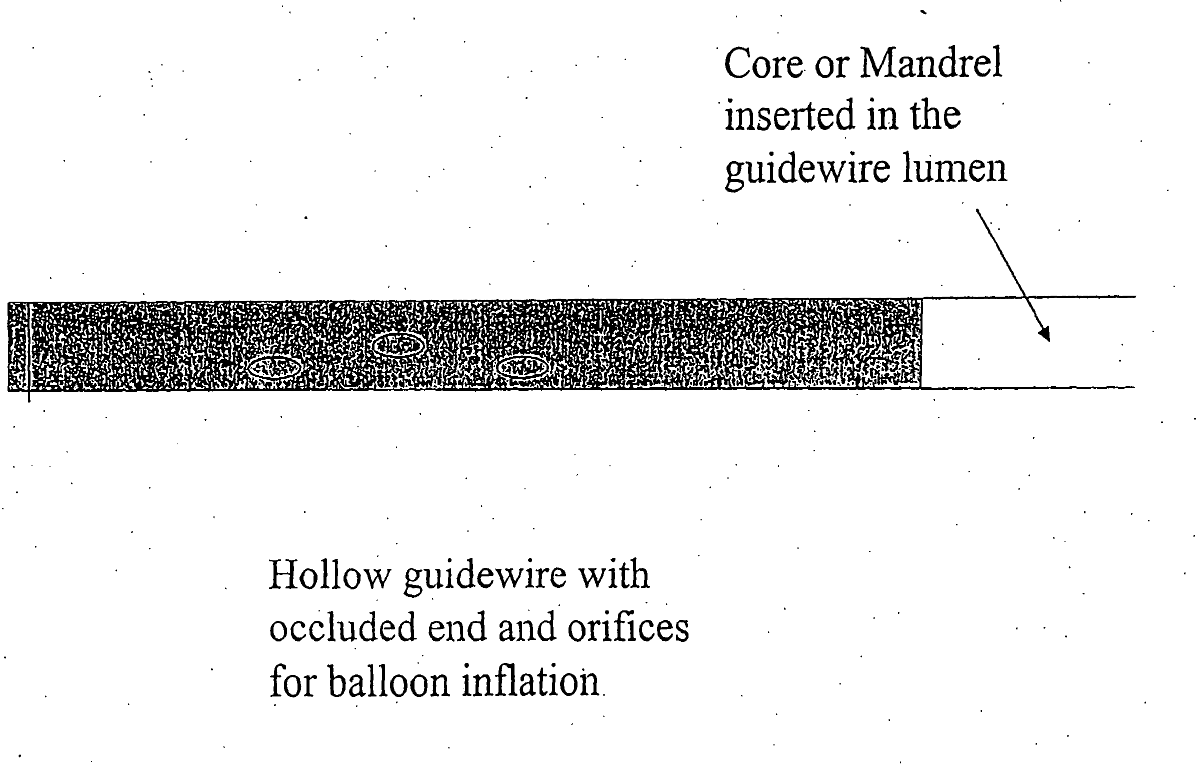

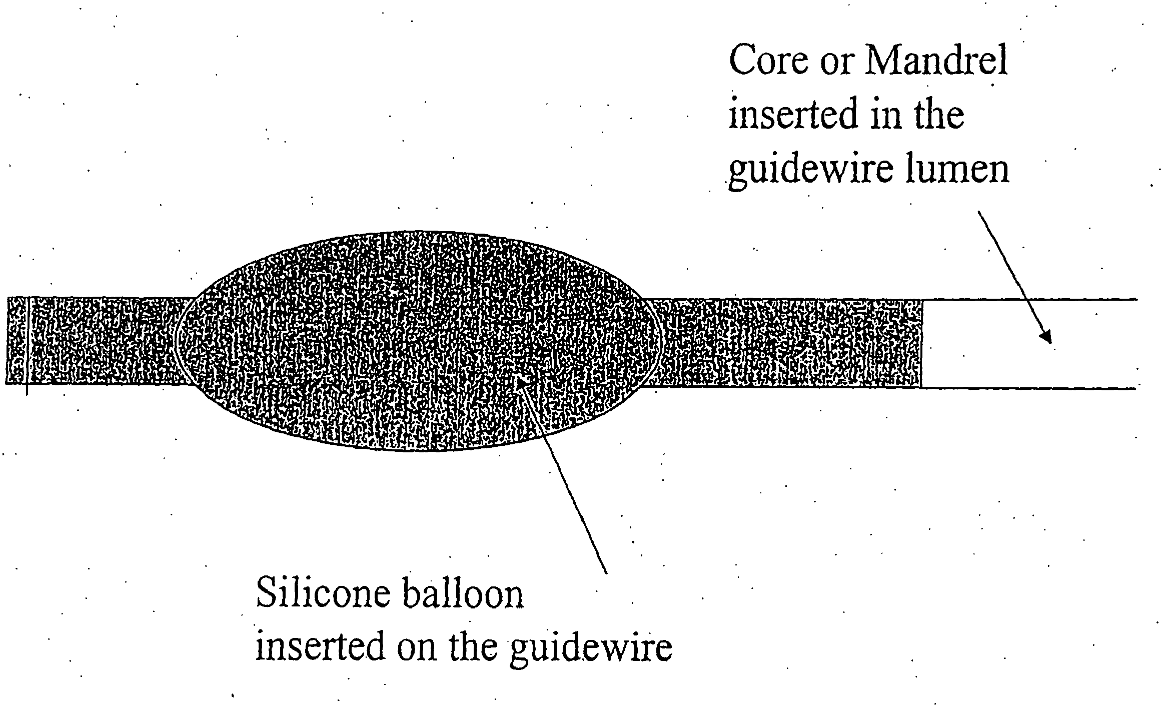

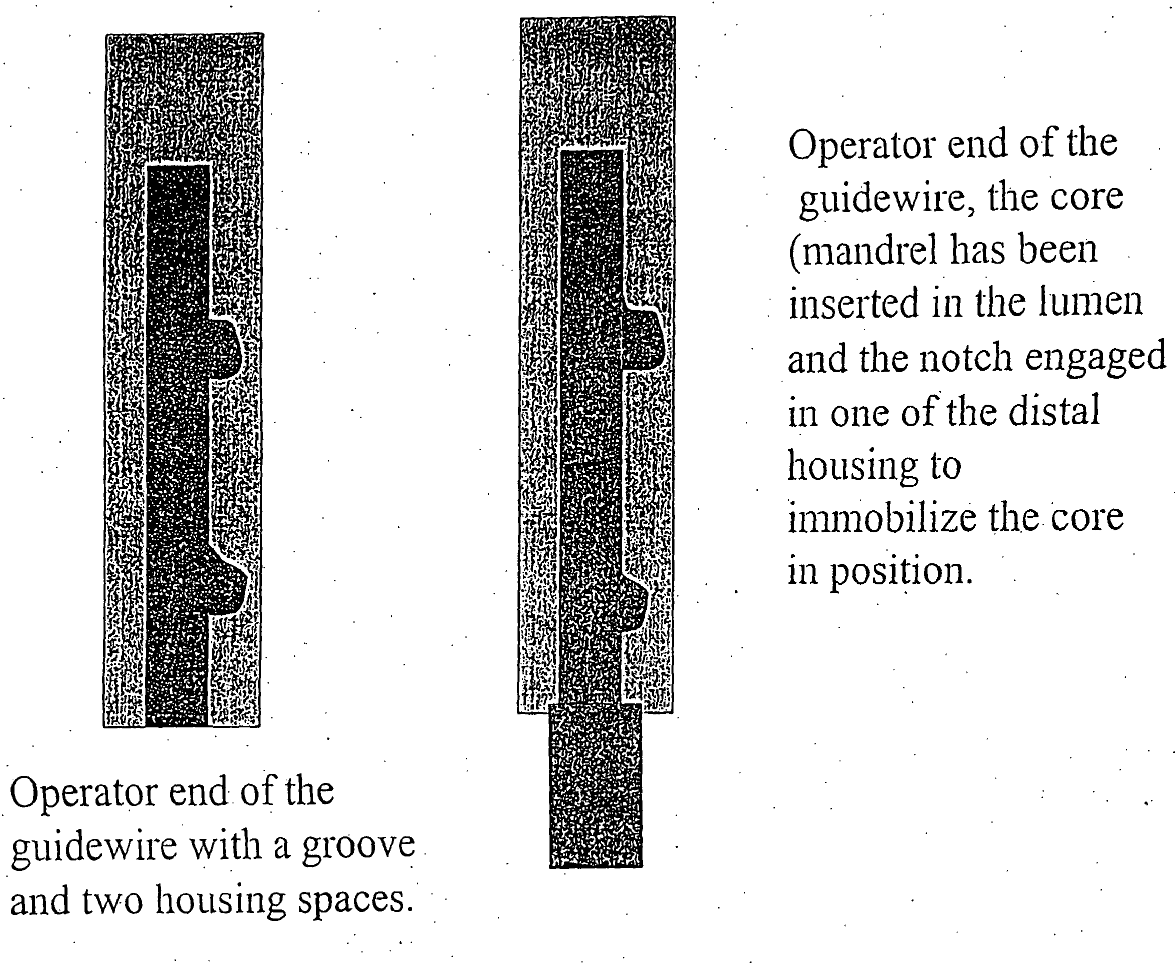

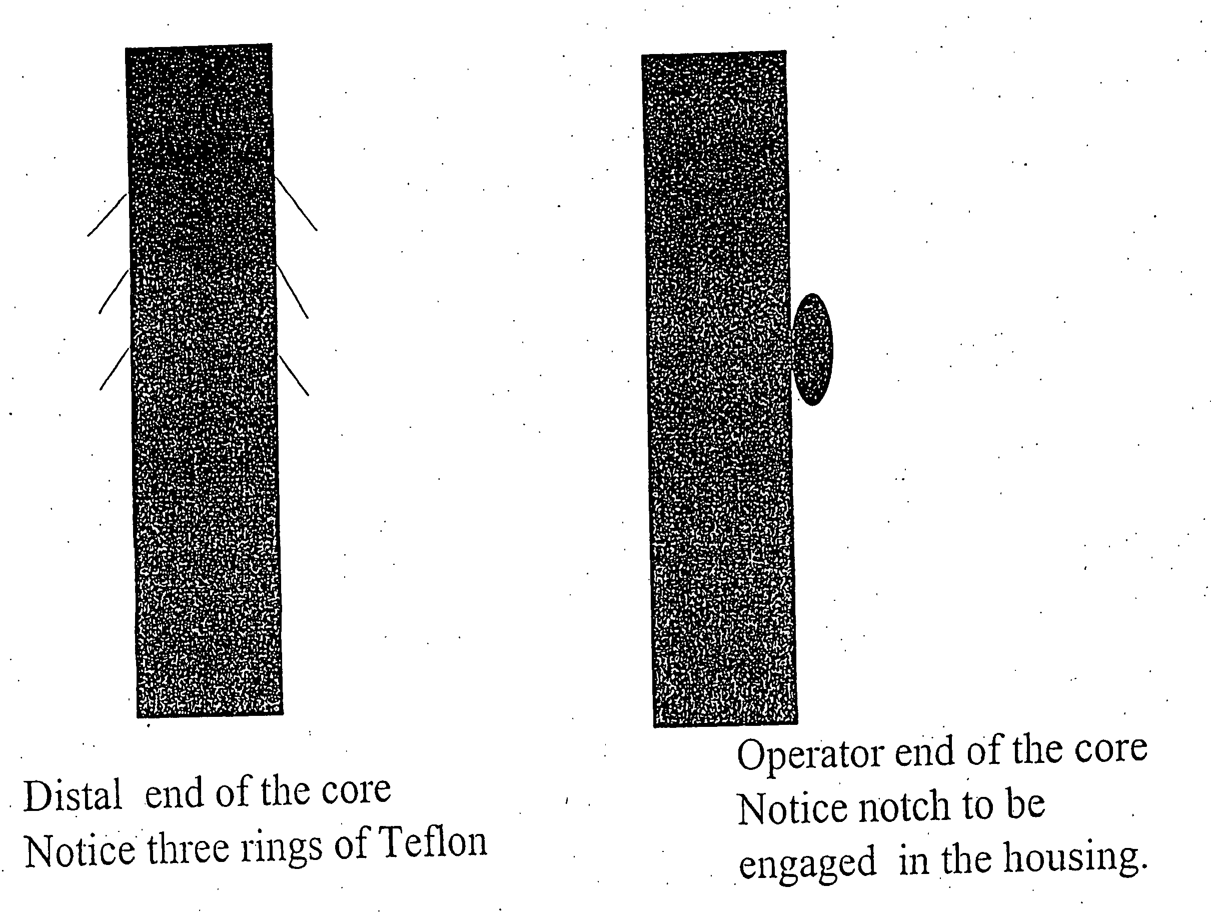

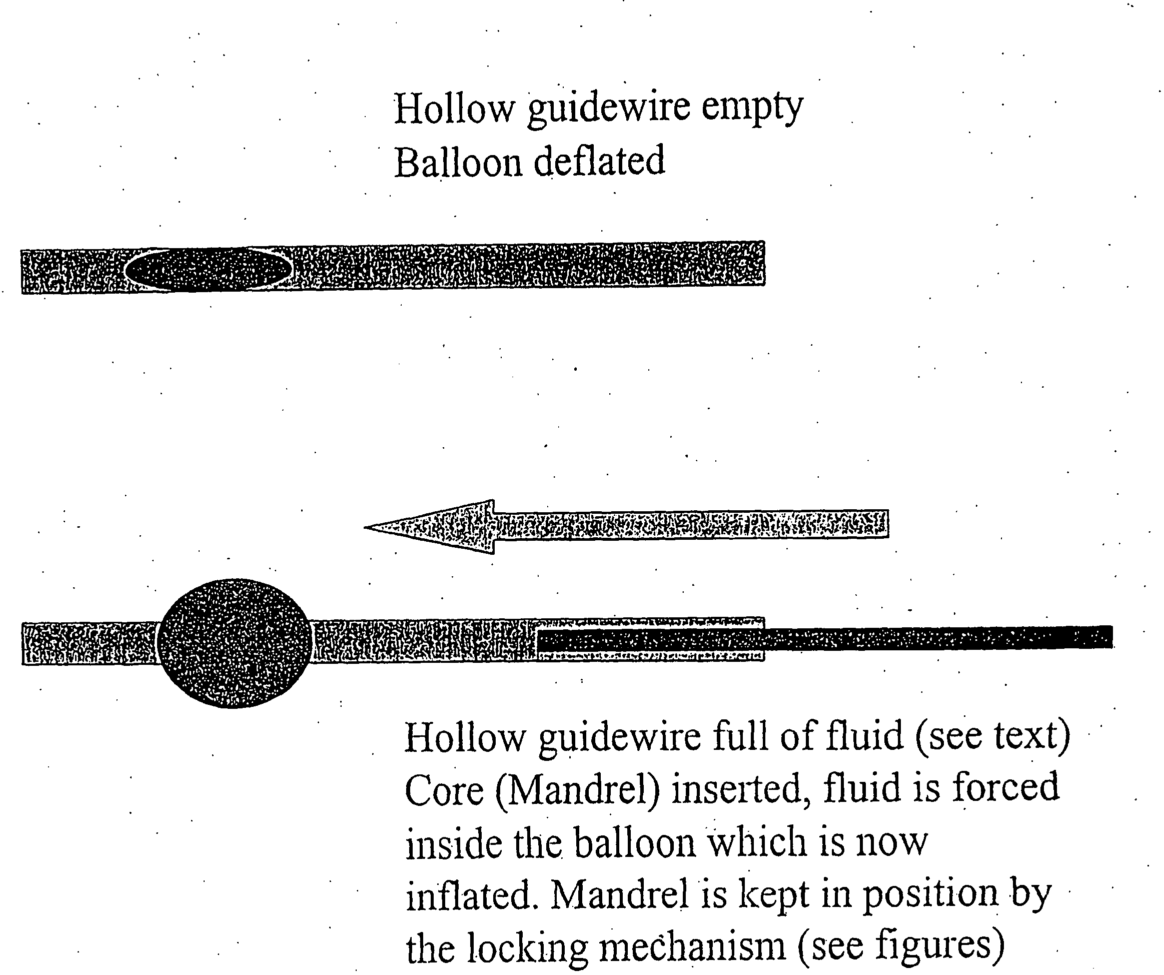

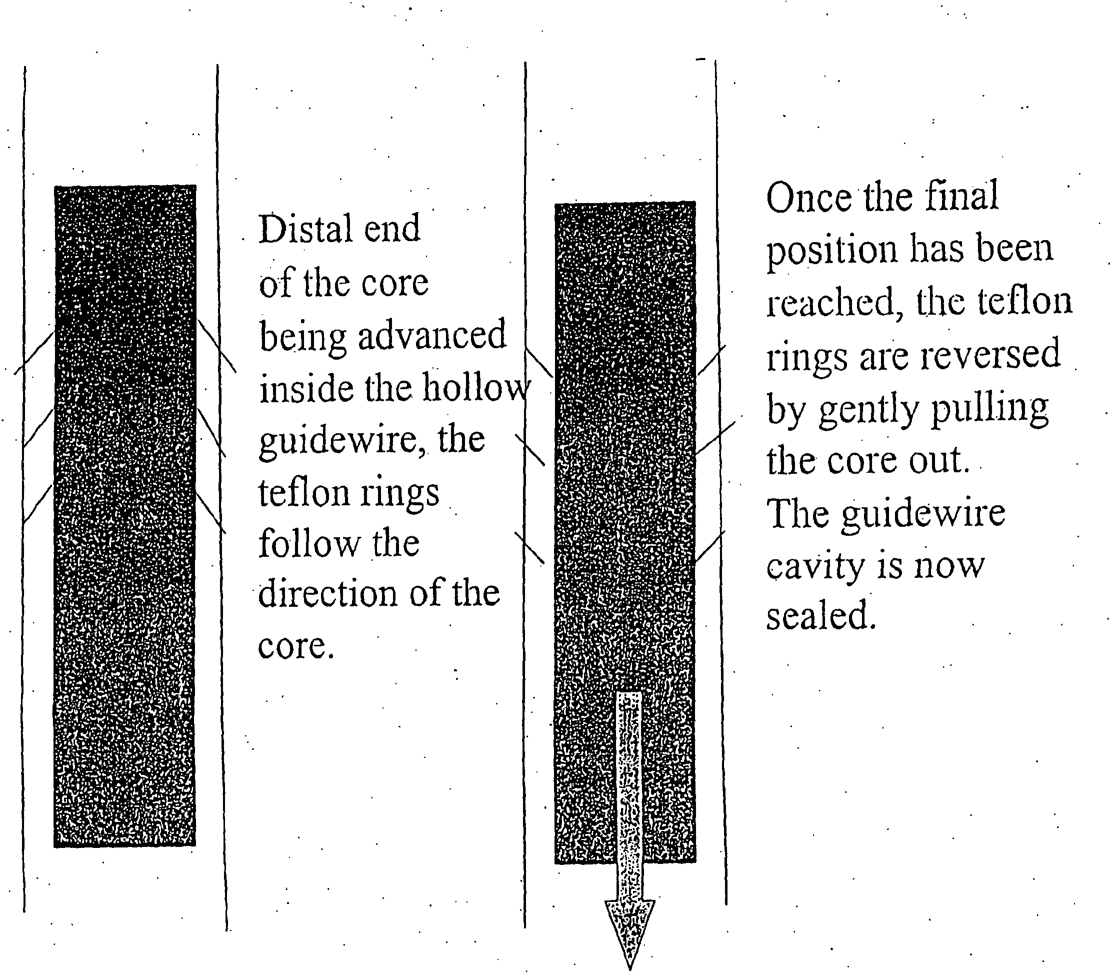

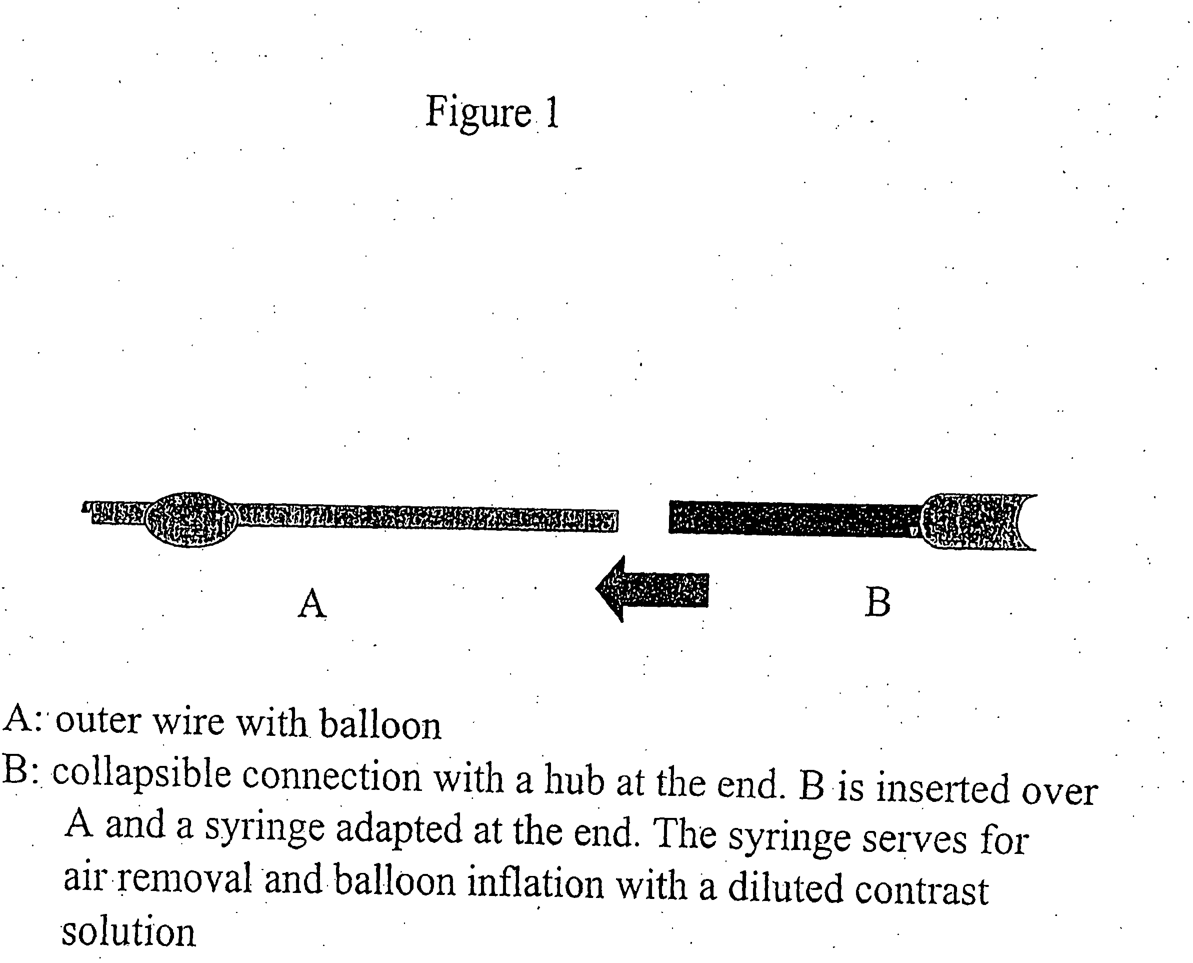

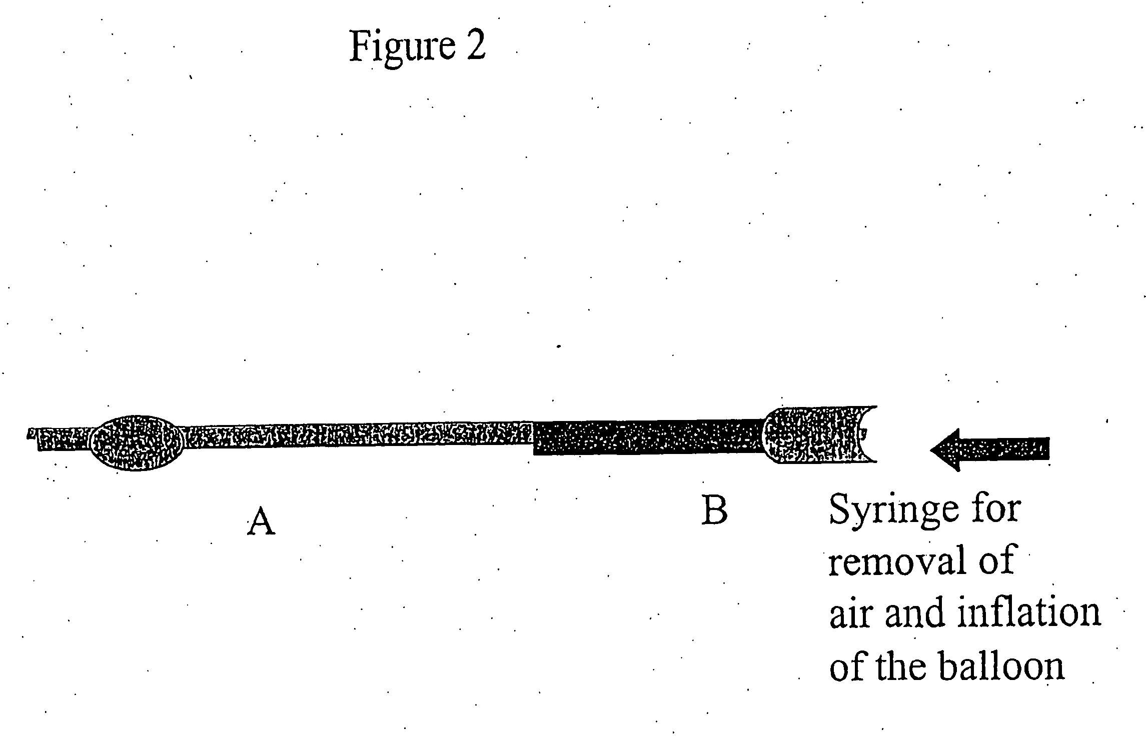

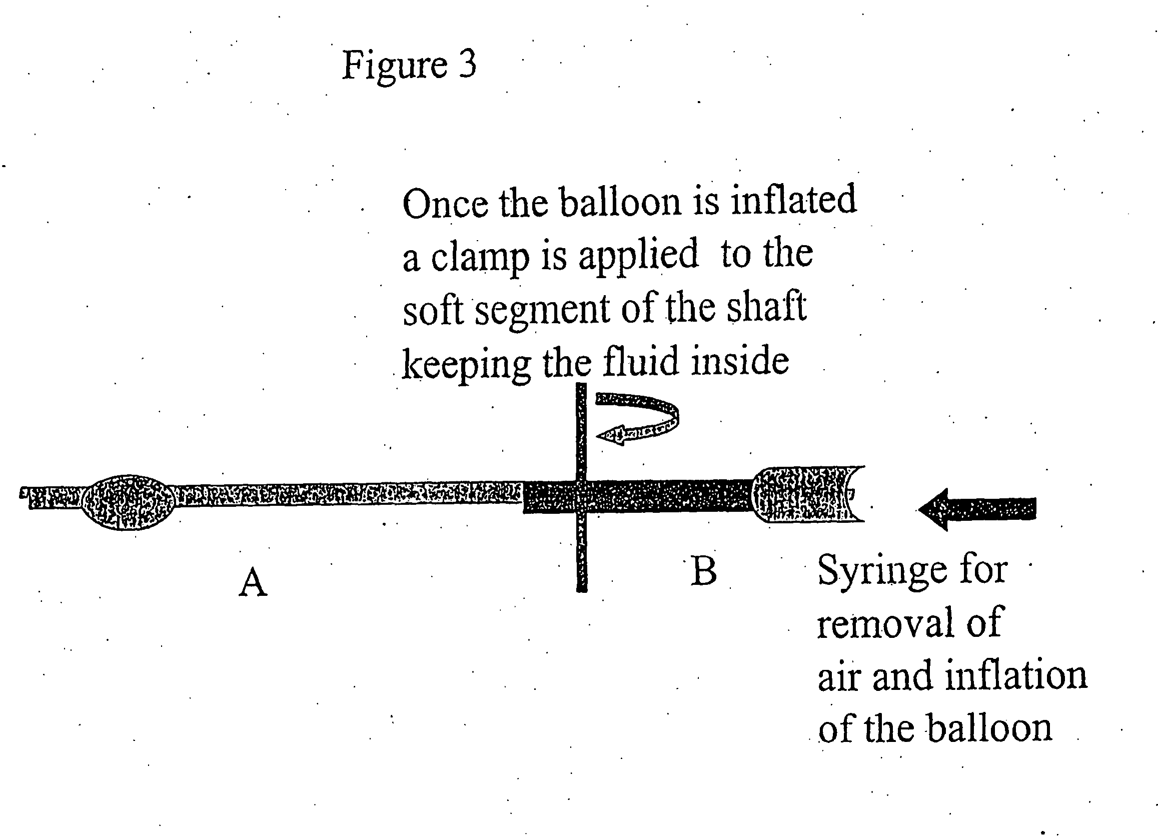

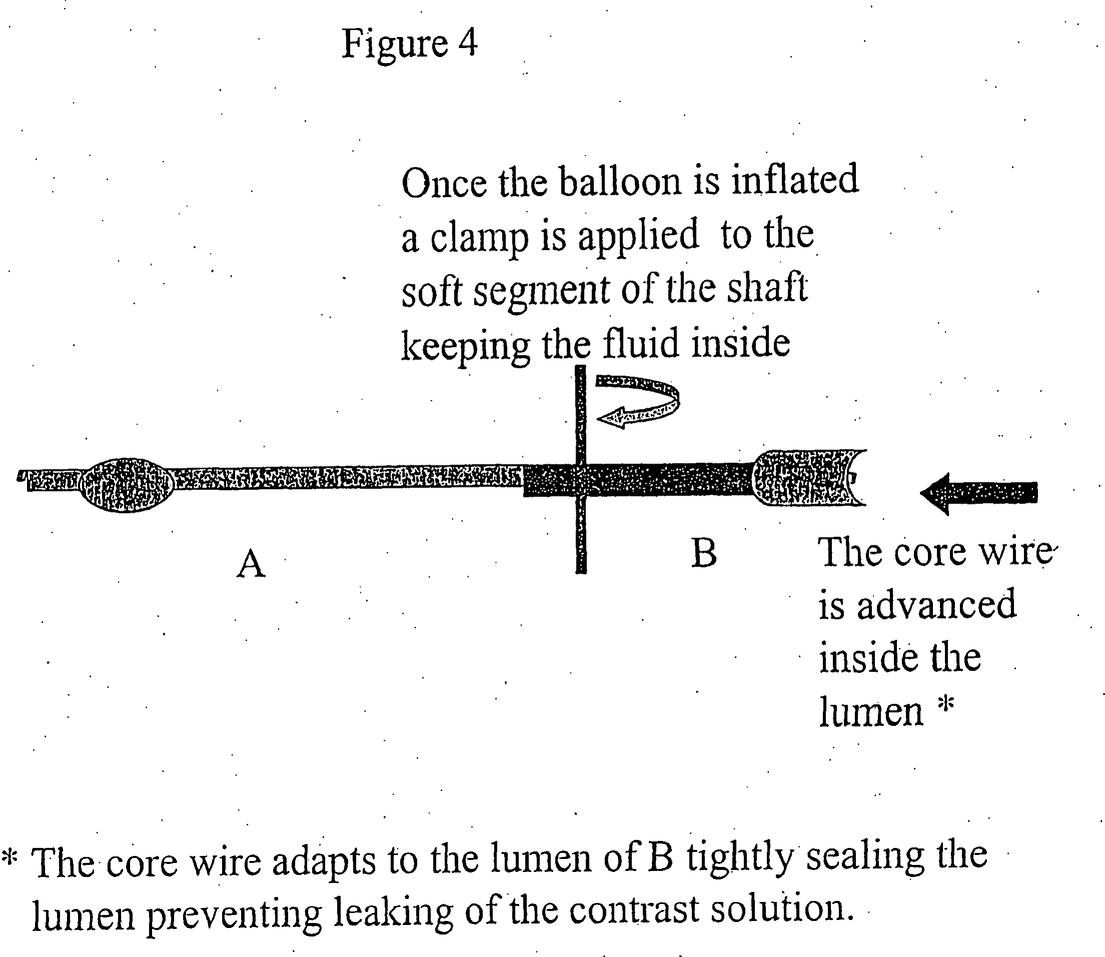

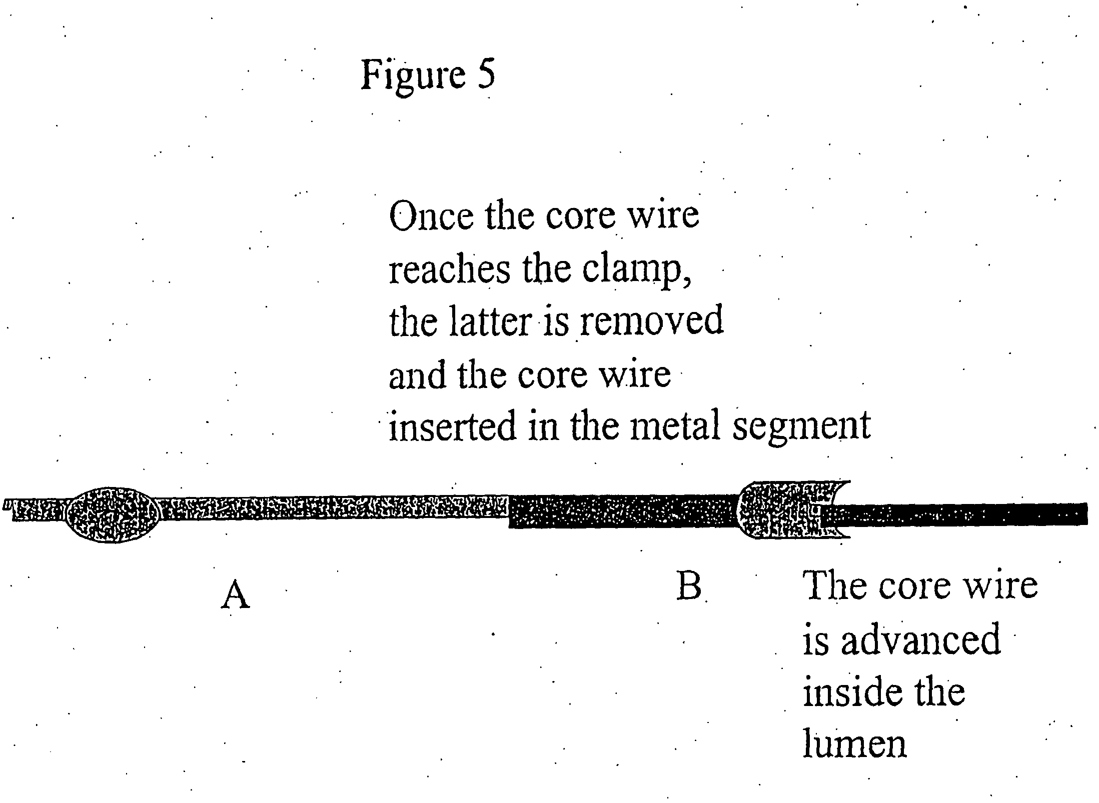

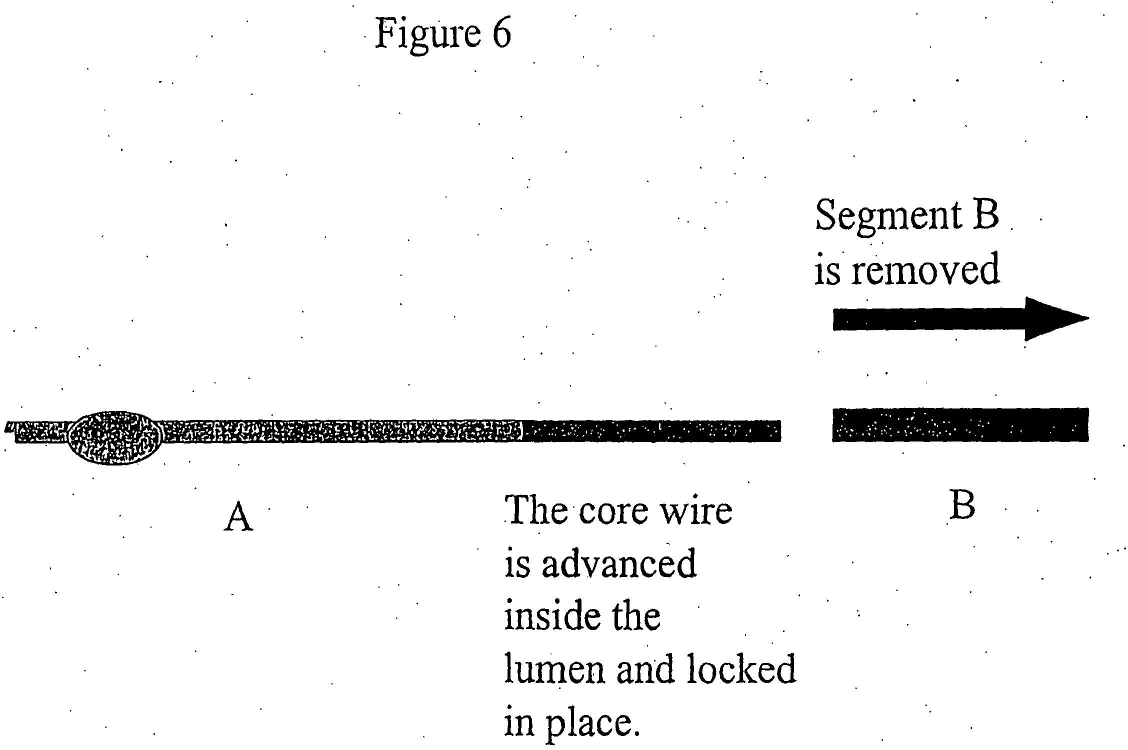

## Claims

1. A device (10) for inflating and deflating a guide wire balloon (20), the device (10) comprising:
a guide wire (14) having a lumen (34), a plurality of orifices (18) extending from the exterior of the guide wire (14) to the lumen (34), and two ends (24,36), the two ends (24,36) comprising a closed end (24) and an open end (36), wherein the lumen (34) provides fluid communication between the orifices (18) and the open end (36);
an inflatable guide wire balloon (20) coupled to the guide wire (14) in communication with the orifices (18);
a core segment (30) disposed within the lumen (34) of the guide wire (14), the core segment (30) configured for removal from the lumen (34) of the guide wire (14), thereby establishing a vacuum in the lumen (34) that facilitates inflation of the guide wire balloon (20);
wherein the guide wire has a low profile topermit a coaxial angioplasty balloon catheter or stent delivery catheter to be exchanged over the guide wire (14) and at least partially over said core segment (30) from the open end (36) to a position just proximal of the balloon (20) without deflating the balloon (20).

2. The device (10) of claim 1, further comprising a solution in communication with the open end (36) of the guide wire (14), the core segment (30) removed from and then advanced into the lumen (34) of the guide wire (14) to inflate the guide wire balloon (20) with the solution.

3. The device (10) of claims 1 or 2, further comprising a locking mechanism configured to lock the core segment (30) in a fixed position within the lumen (34) of the guide wire (14) to maintain the guide wire balloon (20) in an inflated configuration.

4. The device (10) of claim 3, wherein the locking mechanism is disposed within the lumen (34) of the guide wire (14).

5. The device (10) of any one of claims 1 to 4, further comprising a coaxial angioplasty balloon catheter or stent delivery catheter configured for exchange over the guide wire (14) while the guide wire balloon (20) is in the inflated configuration.

6. The device (10) of any one of claims 4, 5 or 6, wherein the locking mechanism further comprises:
a groove (7) disposed on the guide wire (14), the groove (7) extending longitudinally along the guide wire (14) from the open end (36);
a side housing space (8) disposed on the guide wire (14) perpendicular to the groove (7); and
a knob (6) disposed on the core segment (30), the knob (6) configured to engage the housing space (8) and lock the core segment (30) to the guide wire (14).

7. The device (10) of claim 6, further comprising a plurality of side housing spaces (8) disposed on the guide wire (14) perpendicular to the groove (7).

8. The device (10) of any one of claims 1 to 7, further comprising a sealing mechanism configured to prevent fluid leakage around the core segment (30) when the core segment (30) is disposed within the guide wire (14).

9. The device (10) of claim 8, wherein the sealing mechanism comprises a plurality of rings (32) coupled to the core segment (30).

10. The device (10) of claim 8 or 9, wherein the sealing mechanism further comprises a layer of silicone coupled to a distal end portion (50) of the core segment (30) to prevent leakage of fluid around the core segment (30).

## Patentansprüche

1. Vorrichtung (10) zum Aufblasen und Entleeren eines Ballons (20) mit Führungsdraht, wobei die Vorrichtung (10) aufweist:
einen Führungsdraht (14) mit einem Lumen (34), einer Vielzahl von Öffnungen (18), die sich von der Außenseite des Führungsdrahtes (14) zum Lumen (34) erstrecken, und zwei Enden (24, 36), wobei die zwei Enden (24, 36) ein geschlossenes Ende (24) und ein offenes Ende (36) aufweisen, wobei das Lumen (34) eine Fluidverbindung zwischen den Öffnungen (18) und dem offenen Ende (36) bereitstellt;
einen aufblasbaren Ballon (20) mit Führungsdraht, der mit dem Führungsdraht (14) in Verbindung mit den Öffnungen (18) verbunden ist;
ein Kernsegment (30), das innerhalb des Lumens (34) des Führungsdrahtes (14) angeordnet ist, wobei das Kernsegment (30) für ein Entfernen aus dem Lumen (34) des Führungsdrahtes (14) ausgebildet ist, wodurch ein Vakuum im Lumen (34) bewirkt wird, das das Aufblasen des Ballons (20) mit Führungsdraht erleichtert;
wobei der Führungsdraht ein flaches Profil aufweist, damit ein koaxialer Angioplastie-Ballonkatheter oder ein Stentzuführkatheter über dem Führungsdraht (14) und mindestens teilweise über dem Kernsegment (30) vom offenen Ende (36) aus zu einer Position genau proximal vom Ballon (20) ausgetauscht werden kann, ohne dass der Ballon (20) entleert wird.

2. Vorrichtung (10) nach Anspruch 1, die außerdem eine Lösung in Verbindung mit dem offenen Ende (36) des Führungsdrahtes (14) aufweist, bei der das Kernsegment (30) daraus entfernt und danach in das Lumen (34) des Führungsdrahtes (14) transportiert wird, um den Ballon (20) mit Führungsdraht mit der Lösung aufzublasen.

3. Vorrichtung (10) nach Anspruch 1 oder 2, die außerdem einen Verriegelungsmechanismus aufweist, der ausgebildet ist, um das Kernsegment (30) in einer unbeweglichen Position innerhalb des Lumens (34) des Führungsdrahtes (14) zu verriegeln, um den Ballon (20) mit Führungsdraht in einer aufgeblasenen Konfiguration zu halten.

4. Vorrichtung (10) nach Anspruch 3, bei der der Verriegelungsmechanismus innerhalb des Lumens (34) des Führungsdrahtes (14) angeordnet ist.

5. Vorrichtung (10) nach einem der Ansprüche 1 bis 4, die außerdem einen koaxialen Angioplastie-Ballonkatheter oder einen Stentzuführkatheter aufweist, der für einen Austausch über dem Führungsdraht (14) ausgebildet ist, während sich der Ballon (20) mit Führungsdraht in der aufgeblasenen Konfiguration befindet.

6. Vorrichtung (10) nach einem der Ansprüche 4, 5 oder 6, bei der der Verriegelungsmechanismus außerdem aufweist:
eine im Führungsdraht (14) angeordnete Nut (7), wobei sich die Nut (7) in Längsrichtung längs des Führungsdrahtes (14) vom offenen Ende (36) aus erstreckt;
einen seitlichen Gehäusezwischenraum (8), der auf dem Führungsdraht (14) senkrecht zur Nut (7) angeordnet ist; und
einen Knopf (6), der am Kernsegment (30) angeordnet ist, wobei der Knopf (6) ausgebildet ist, um mit dem Gehäusezwischenraum (8) in Eingriff zu kommen und das Kernsegment (30) am Führungsdraht (14) zu verriegeln.

7. Vorrichtung (10) nach Anspruch 6, die außerdem eine Vielzahl von seitlichen Gehäusezwischenräumen (8) aufweist, die auf dem Führungsdraht (14) senkrecht zur Nut (7) angeordnet sind.

8. Vorrichtung (10) nach einem der Ansprüche 1 bis 7, die außerdem einen Abdichtungsmechanismus aufweist, der ausgebildet ist, um einen Fluidaustritt um das Kernsegment (30) herum zu verhindern, wenn das Kernsegment (30) innerhalb des Führungsdrahtes (14) angeordnet wird.

9. Vorrichtung (10) nach Anspruch 8, bei der der Abdichtungsmechanismus eine Vielzahl von Ringen (32) aufweist, die mit dem Kernsegment (30) verbunden sind.

10. Vorrichtung (10) nach Anspruch 8 oder 9, bei dem der Abdichtungsmechanismus außerdem eine Schicht von Silikon aufweist, die mit einem distalen Endabschnitt (50) des Kernsegmentes (30) verbunden ist, um einen Austritt des Fluids um das Kernsegment (30) herum zu verhindern.

## Revendications

1. Dispositif (10) pour gonfler et dégonfler un ballonnet de fil-guide (20), le dispositif (10) comprenant :
un fil-guide (14) comportant une lumière (34), plusieurs orifices (18), s'étendant de l'extérieur du fil-guide (14) vers la lumière (34), et deux extrémités (24, 36), les deux extrémités (24, 36) comprenant une extrémité fermée (24) et une extrémité ouverte (36), dans lequel la lumière (34) établit une communication de fluide entre les orifices (18) et l'extrémité ouverte (36) ;
un ballonnet de fil-guide gonflable (20), accouplé au fil-guide (14), en communication avec les orifices (18) ;
un segment de noyau (30), agencé dans la lumière (34) du fil-guide (14), le segment de noyau (30) étant configuré de sorte à être retiré à partir de la lumière (34) du fil-guide (14), établissant ainsi un vide dans la lumière (34) facilitant le gonflement du ballonnet du fil-guide (20) ;
dans lequel le fil-guide a un profil bas pour permettre l'échange d'un cathéter à ballonnet d'angioplastie coaxial ou d'un cathéter de pose d'un stent au-dessus du fil-guide (14) et au moins en partie au-dessus dudit segment de noyau (30), de l'extrémité ouverte (36) vers une position juste proximale par rapport au ballonnet (20), sans dégonfler le ballonnet (20) ;

2. Dispositif (10) selon la revendication 1, comprenant en outre une solution en communication avec l'extrémité ouverte (36) du fil-guide (14), le segment de noyau (30) étant retiré de la lumière (34) du fil-guide (14) avant d'y être avancé, afin de gonfler le ballonnet du fil-guide (20) avec la solution.

3. Dispositif (10) selon les revendications 1 ou 2, comprenant en outre un mécanisme de verrouillage, configuré de sorte à verrouiller le segment de noyau (30) dans une position fixe dans la lumière (34) du fil-guide (14), afin de maintenir le ballonnet du fil-guide (20) dans une configuration gonflée.

4. Dispositif (10) selon la revendication 3, dans lequel le mécanisme de verrouillage est agencé dans la lumière (34) du fil-guide (14).

5. Dispositif (10) selon l'une quelconque des revendications 1 à 4, comprenant en outre un cathéter à ballonnet d'angioplastie coaxial ou un cathéter de pose d'un stent, configuré pour un échange au-dessus du fil-guide (14) pendant que le ballonnet du fil-guide (20) se trouve dans la configuration gonflée.

6. Dispositif (10) selon l'une quelconque des revendications 4, 5 ou 6, dans lequel le mécanisme de verrouillage comprend en outre :
une rainure (7) agencée sur le fil-guide (14), la rainure (7) s'étendant longitudinalement le long du fil-guide (14) à partir de l'extrémité ouverte (36) ;
un espace de logement latéral (8), agencé sur le fil-guide (14), de manière perpendiculaire à la rainure (7) ; et
un bouton (6) agencé sur le segment de noyau (30), le bouton (6) étant configuré de sorte à s'engager avec l'espace du logement (8) et à verrouiller le segment de noyau (30) sur le fil-guide (14).

7. Dispositif (10) selon la revendication 6, comprenant en outre plusieurs espaces de logement latéraux (8) agencés sur le fil-guide (14), de manière perpendiculaire à la rainure (7).

8. Dispositif (10) selon l'une quelconque des revendications 1 à 7, comprenant en outre un mécanisme d'étanchéité, configuré de sorte à empêcher une fuite du fluide autour du segment de noyau (30) lorsque le segment de noyau (30) est agencé dans le fil-guide (14).

9. Dispositif (10) selon la revendication 8, dans lequel le mécanisme d'étanchéité comprend plusieurs bagues (32) accouplées au segment de noyau (30).

10. Dispositif (10) selon les revendications 8 ou 9, dans lequel le mécanisme d'étanchéité comprend en outre une couche de silicone accouplée à une partie d'extrémité distale (50) du segment de noyau (30), pour empêcher une fuite du fluide autour du segment de noyau (30).
